# EUROPEAN PATENT SPECIFICATION

(11) **EP 2 077 987 B1**
(45) Date of publication and mention of the grant of the patent: **24.11.2010**
(21) Application number: 07839756.9
(22) Date of filing: 24.10.2007
(51) Int. Cl.: C07C 69/734, C12P 41/00

(54) **MANDELIC ACID DERIVATIVES AND PREPARATION THEREOF**
MANDELSÄUREDERIVATE UND DEREN HERSTELLUNG
DÉRIVÉS DE L'ACIDE MANDÉLIQUE ET PRÉPARATION DE CEUX-CI

(30) Priority: 25.10.2006 US 854347 P
(43) Date of publication of application: 15.07.2009
(73) Proprietor: TransTech Pharma, Inc., High Point, NC 27265 (US)
(72) Inventor: POLISETTI, Dharma R., High Point, NC 27265 (US); YOKUM, Thomas Scott, Greensboro, NC 27407 (US); GUZEL, Mustafa, Jamestown, NC 27282 (US); BONDLELA, Muralidhar, Greensboro, NC 27410 (US); CHRISTEN, Daniel P., Jamestown, NC 27282 (US)
(74) Representative: Russell, Lindsey
(86) International application number: PCT/US2007/022535
(87) International publication number: WO 2008/051563

(56) References cited:
- DE-A1- 4 037 251
- DE-A1- 4 414 273

## Description

### FIELD OF THE INVENTION

The invention relates to compounds, compositions, methods of using and processes for producing optically active α-hydroxy compounds using a combination of a chemical and biochemical methods.

### BACKGROUND OF THE INVENTION

Optically active compounds are useful chemical compounds as starting materials or as intermediates for physiologically active materials of medical supplies, agricultural chemicals and the like. From optically active α-hydroxyesters, optically active haloesters (B. J. Lee, et al., Tetrahedron, 23, 359(1967), optically active glycols (V. Prelog, et al., Helv. Chim, Acta. 37, 234(1954)), optically active epoxides (K. Mori, et al., Tetrahedron, 35, 933(1979)) and other compounds of similar structure, one can generate other optically active compounds that are useful.

Optically active compounds can be used for optical resolving agents. In one example, optically active compounds can be used for optically resolving agents of medical or agricultural supplies such as 2-amino-1-butanol which is a starting material of the antituberculous drug ethambutol, diltiazem hydrochloride which is a coronary vasodilator and tetramizol, which is effective as an anthelmintic (see, for example, Japanese Patent Publication No. 61-52812, and Japanese Patent Unexamined Publication Nos. 58-32872 and 62-192388). In another example, optically active compounds are useful for optically resolving agents of α-amino acids such as alanine, phenyl alanine, methionine, cysteine and the other similar amino acids (see, for example, Japanese Patent Unexamined Publication Nos. 55-57545 and 60-32752, Japanese Patent Publication No. 58-1105, and Japanese Patent Unexamined Publication Nos. 59-181244, 57-193448 and 59-51239).

Optically pure compounds can be used as starting materials or intermediates useful to synthesize optically pure therapeutic agents. For example, optically active homophenylalanine can be used to make 2-amino-4-phenylbutanoates derivatives such as enalapril which is an ACE inhibitor (angiotensin converting enzyme inhibitor) and the like. (See, for example, H. Urbach and R. Henning, Tetrahedron Lett., 25, 1143(1984)).

US Patent No. 5,248,610 provides an example of how to make optically active compounds by the use of a lipase. Researchers have also generated optically active compounds through the asymmetric reduction of α-ketoesters using microorganism or baker's yeast. (see, for example, K. Nakamura et al., J. Org. Chem., 53, 2589(1988), K. Nakamura et al., Tetrahedron Letters., 29, 2453(1988), Japanese Patent Unexamined Publication No. 62-61587). Others have chemically reduced α -ketoesters to generate optically active α -hydroxyesters by using arylglyoxylic acid. These methods produced α -hydroxyesters with a 2.4-9.7% ee. (also see, for example, I. Takahashi et al., Chem. Pharm. Bull., 33 3571(1985)). Other researchers have obtained optically active α -hydroxyesters through asymmetric reduction of ketones with an organic boron compound. (see, for example, H. C. Brown et al., J. Org. Chem., 53, 1231(1988)).

Thus, there is a need for methods to access a broad array of optically active compounds.

### BRIEF SUMMARY OF THE INVENTION

The present invention relates to compounds, compositions, methods of using and processes for producing optically active α-hydroxy derivatives using a combination of chemical and biochemical methods. In an embodiment, the process comprises reacting an ester compound with a racemic compound in the presence of an enzyme to obtain optically active α-hydroxy derivatives that can be used in compositions, that may be useful for starting materials for physiologically active materials, functional materials and the like, and that may be used in methods to resolve other racemic compounds.

Formula I shows an exemplary embodiment of the optically active α-hydroxy derivatives that can be accessed using methods of the present invention: 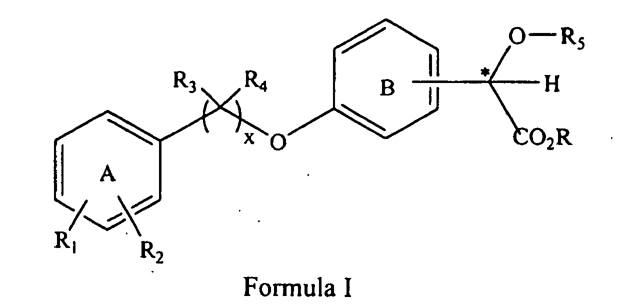 wherein
R₁ and R₂ are independently selected from the group consisting of: halo, hydroxy, cyano, C₁₋₄alkyl, C₁₋₄alkoxy, C₁₋₄haloalkyl, C₁₋₄haloalkoxy, wherein at least one of R₁ and R₂ is halo,
R₃ and R₄ are each independently hydrogen, cyano, C₁₋₆alkyl, C₁₋₆alkoxy, C₁₋₆haloalkyl, C₁₋₆haloalkoxy, phenyl, or taken together R₃ and R₄ are oxo; x is 1, 2, 3, 4, 5, or 6;
R₅ is hydrogen or C₁₋₁₈ acyl;
wherein phenyl ring A and B are further optionally substituted with one or more of halo, C₁₋₄ alkyl, C₁₋₄ alkoxy, C₁₋₄ haloalkyl, and C₁₋₄ haloalkoxy; and
R is hydrogen, C₁₋₆ alkyl, an alkali metal ion.

### DETAILED DESCRIPTION OF THE INVENTION

The present invention relates to compounds, compositions and methods of making and using compounds as shown throughout this application. For the purposes of this invention, whenever compounds are referred to in this invention, it is contemplated and therefore part of the scope of the present invention that it also includes the compositions, and methods of making and methods of using the compounds.

In one aspect, the present invention relates to the compound of Formula I (and compositions, methods of making and methods of using the compounds associated with this compound): 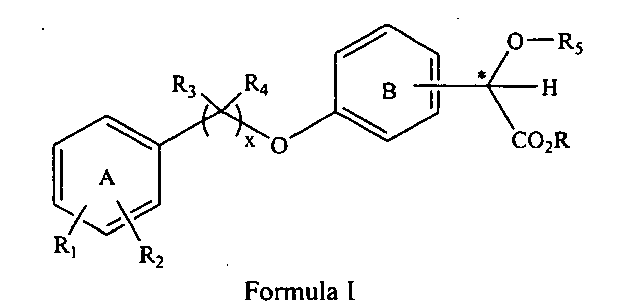 wherein
R₁ and R₂ are independently selected from the group consisting of: halo, hydroxy, cyano, C₁₋₄alkyl, C₁₋₄alkoxy, C₁₋₄haloalkyl, C₁₋₄haloalkoxy, wherein at least one of R₁ and R₂ is halo,
R₃ and R₄ are each independently hydrogen, cyano, C₁₋₆ alkyl, C₁₋₆alkoxy, C₁₋₆haloalkyl, C₁₋₆haloalkoxy, phenyl, or taken together R₃ and R₄ are oxo ;
x is 1, 2, 3, 4, 5, or 6;
R₅ is hydrogen or C₁₋₁₈ acyl;
wherein phenyl ring A and B are further optionally substituted with one or more of halo, C₁₋₄alkyl, C₁₋₄ alkoxy, C₁₋₄haloalkyl, and C₁₋₄haloalkoxy; and
R is hydrogen, C₁₋₆ alkyl, or an alkali metal ion.

In an alternate embodiment, R₁ and R₂ are both halo. The halo is iodo, fluoro, chloro, or bromo. In an alternate embodiment, both R₁ and R₂ are chloro.

In an alternate embodiment, R₅ is hydrogen or acetyl.

In an embodiment, x is 1 or 2. In an alternate embodiment, the carbon at * is in the R configuration or alternatively, the carbon at * is in the S configuration.

In a further embodiment, R₁ and R₂ are both chloro, R₅ is hydrogen or acetyl, and x is 1. In a further embodiment, R₁ is halo and R₂ is C₁₋₄ alkyl or C₁₋₄ haloalkyl.

In another embodiment, R₁ and R₂ are independently selected from the group consisting of hydrogen, chloro, fluoro, -CF₃, and -OCF₃, wherein at least one of R₁ and R₂ is not hydrogen.

In a further embodiment, R is methyl.

In another embodiment, the R₃ and R₄ are hydrogen and x = 1.

In another embodiment, phenyl ring A and B are not further substituted.

In another embodiment, the compound of Formula I is above 95% ee. In another embodiment, the compound of Formula I is above 99% ee.

In an alternate embodiment, the present invention relates to compounds of Formula IA 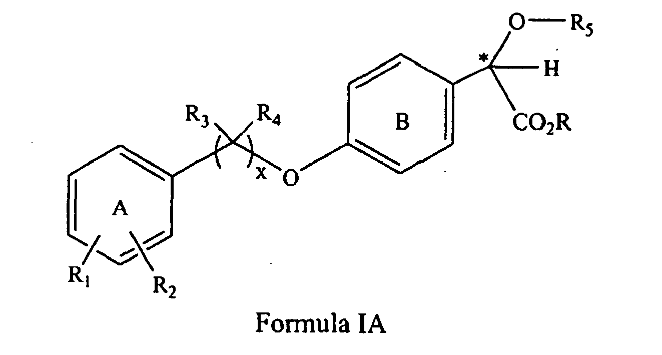 wherein all of R₁, R₂, R₃, R₄, R₅, R and x are defined as above for the compound of Formula I.

As above, in an embodiment, R₁ and R₂ are both halo. The halo is iodo, fluoro, chloro, or bromo. In an alternate embodiment, both R₁ and R₂ are chloro. In a further embodiment, R₁ is halo and R₂ is C₁₋₄ alkyl or C₁₋₄ haloalkyl.

In an alternate embodiment, R₅ is hydrogen or acetyl.

In an embodiment, x is 1 or 2. In an alternate embodiment, the carbon at * is in the R configuration or alternatively, the carbon at * is in the S configuration.

In a further embodiment, R₁ and R₂ are both chloro, R₅ is hydrogen or acetyl, and x is 1.

In another embodiment, R₁ and R₂ are independently selected from the group consisting of hydrogen, chloro, fluoro, -CF₃, and -OCF₃, wherein at least one of R₁ and R₂ is not hydrogen.

In a further embodiment, R is methyl.

In another embodiment, the R₃ and R₄ are hydrogen and x = 1.

In another embodiment, phenyl ring A and B are not further substituted.

In an embodiment, the compound of Formula IA is above 95% ee. In another embodiment, the compound of Formula IA is above 99% ee.

In an alternate embodiment, the present invention relates to compounds of Formula IB 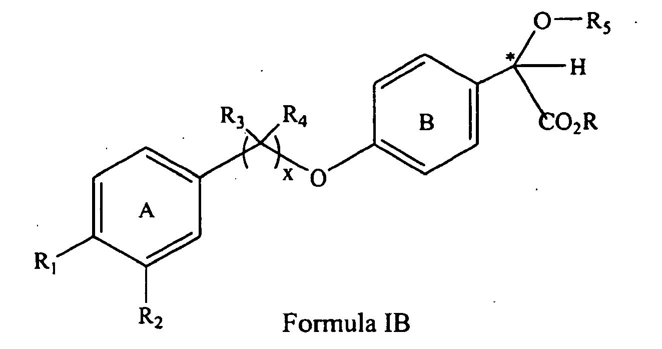 wherein all of R₁, R₂, R₃, R₄, R ₅, R and x are defined as above for the compound of Formula I.

As above, in an embodiment, R₁ and R₂ are both halo. The halo is iodo, fluoro, chloro, or bromo. In an alternate embodiment, both R₁ and R₂ are chloro. In a further embodiment, R₁ is halo and R₂ is C₁₋₄ alkyl or C₁₋₄ haloalkyl.

In an alternate embodiment, R₅ is hydrogen or acetyl.

In an embodiment, x is 1 or 2. In an alternate embodiment, the carbon at * is in the R configuration or alternatively, the carbon at * is in the S configuration.

In a further embodiment, R₁ and R₂ are both chloro, R₅ is hydrogen or acetyl, and x is 1.

In another embodiment, R₁ and R₂ are independently selected from the group consisting of hydrogen, chloro, fluoro, -CF₃, and -OCF₃, wherein at least one of R₁ and R₂ is not hydrogen.

In a further embodiment, R is methyl.

In another embodiment, the R₃ and R₄ are hydrogen and x = 1.

In another embodiment, phenyl ring A and B are not further substituted.

In an embodiment, the compound of Formula IB is above 95% ee. In another embodiment, the compound of Formula IB is above 99% ee.

As used herein, "haloalkyl" refers to a straight or branched chain hydrocarbon substituted with at least one halogen atom and optionally substituted at the remaining positions with a halogen atom. A haloalkyl group may be substituted with one or more types of halogen atoms. Examples of "haloalkyl" as used herein include, but are not limited to, a trifluoromethyl group, a 2,2,2-trifluoroethyl group, and the like.

As used herein, "haloalkoxy" refers to a straight or branched chain alkoxy group substituted with at least one halogen atom and optionally substituted at the remaining positions with a halogen atom. A haloalkyloxy group may be substituted with one or more types of halogen atoms. Examples of "haloalkyloxy" as used herein include, but are not limited to, a trifluoromethoxy group, a 2,2,2-trifluoroethoxy group, and the like.

As used herein, "C₁₋₁₈ acyl" refers to the group RₐC(O)-, where Rₐ is either hydrogen or a hydrocarbon containing 1 to 17 carbons being either straight or branched and optionally having one or more units of unsaturation.

As used herein, an "alkali metal ion" refers to metal ions in Group IA and IIA of the periodic chart. In an embodiment, an alkali metal ion may be selected from the group consisting of Na⁺ and K⁺.

As used herein, "pharmaceutically acceptable salts" of the compounds of the present invention, where a basic or acidic group is present in the structure, are also included within the scope of the invention. The term "pharmaceutically acceptable salts" refers to non-toxic salts of the compounds of this invention which are generally prepared by reacting the free base with a suitable organic or inorganic acid or by reacting the acid with a suitable organic or inorganic base. Representative salts include the following salts: Acetate, Benzenesulfonate, Benzoate, Bicarbonate, Bisulfate, Bitartrate, Borate, Bromide, Calcium Edetate, Camsylate, Carbonate, Chloride, Clavulanate, Citrate, Dihydrochloride, Edetate, Edisylate, Estolate, Esylate, Fumarate, Gluceptate, Gluconate, Glutamate, Glycollylarsanilate, Hexylresorcinate, Hydrabamine, Hydrobromide, Hydrocloride, Hydroxynaphthoate, Iodide, Isethionate, Lactate, Lactobionate, Laurate, Malate, Maleate, Mandelate, Methanesulfonate, Methylbromide, Methylnitrate, Methylsulfate, Monopotassium Maleate, Mucate, Napsylate, Nitrate, N-methylglucamine, Oxalate, Pamoate (Embonate), Palmitate, Pantothenate, Phosphate/diphosphate, Polygalacturonate, Potassium, Salicylate, Sodium, Stearate, Subacetate, Succinate, Tannate, Tartrate, Teoclate, Tosylate, Triethiodide, Trimethylammonium and Valerate. When an acidic substituent is present, such as-COOH, there can be formed the ammonium, morpholinium, sodium, potassium, barium, calcium salt, and the like, for use as the dosage form. When a basic group is present, such as amino or a basic heteroaryl radical, such as pyridyl, an acidic salt, such as hydrochloride, hydrobromide, phosphate, sulfate, trifluoroacetate, trichloroacetate, acetate, oxlate, maleate, pyruvate, malonate, succinate, citrate, tartarate, fumarate, mandelate, benzoate, cinnamate, methanesulfonate, ethanesulfonate, picrate and the like, and include acids related to the pharmaceutically-acceptable salts listed in the Journal of Pharmaceutical Science, 66, 2 (1977) p. 1-19.

The compounds of the present invention may have use as a starting material to make other chiral containing compounds such as physiologically active materials of medical supplies or agricultural chemicals or as chiral resolving agents. In an embodiment, the optically pure compounds of the present invention can be incorporated into larger compounds.

In another aspect, the present invention relates to a method comprising reacting a compound of Formula X 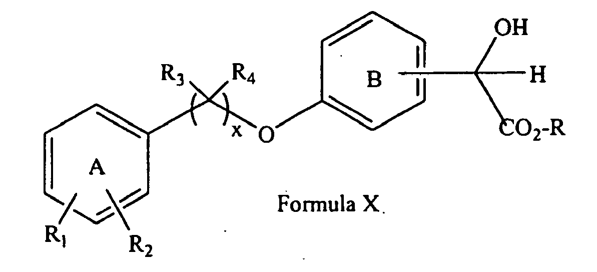 wherein
R₁ and R₂ are independently selected from the group consisting of: halo, hydroxy, cyano, C₁₋₄alkyl, C₁₋₄alkoxy, C₁₋₄haloalkyl, C₁₋₄haloalkoxy, wherein at least one of R₁ and R₂ is halo,
R₃ and R₄ are each independently hydrogen, cyano, C₁₋₆alkyl, C₁₋₆alkoxy, C₁₋₆haloalkyl, C₁₋₆ haloalkoxy, phenyl, or taken together R₃ and R₄ are oxo ;
x is 1, 2, 3, 4, 5, or 6;
wherein phenyl ring A and B are further optionally substituted with one or more of halo, C₁₋₄alkyl, C₁₋₄ alkoxy, C₁₋₄ haloalkyl, and C₁₋₄ haloalkoxy; and
R is C₁₋₆ alkyl,
with an enzyme and an ester compound to form a reaction mixture;
generating a compound of Formula XI 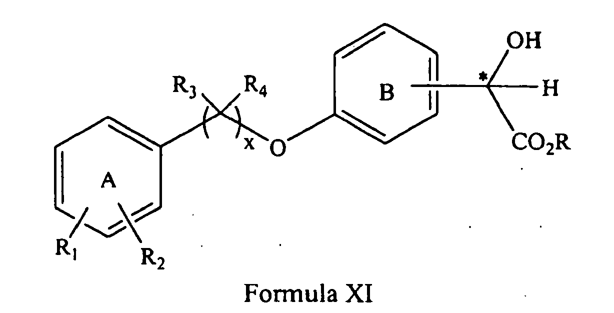 wherein R₁, R₂ R₃, R₄, R and x are as described above for the compound of Formula X, wherein the carbon with the * present is in the R or S configuration;
and
generating a compound of Formula XII 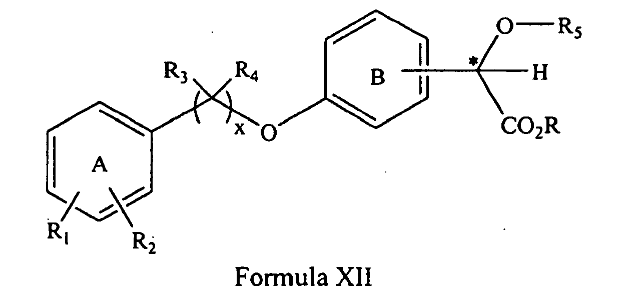 wherein R₁, R₂ R₃, R₄, R and x are as described above for the compound of Formula X and R₅ is a C₁₋₁₈ acyl group, and wherein the carbon with the * present is in the opposite configuration as the compound of Formula XI.

In an embodiment of the method, R₁ and R₂ are both halo. The halo is iodo, fluoro, chloro, or bromo. In an alternate embodiment, both R₁ and R₂ are chloro.

In another embodiment, x is 1 or 2.

In a further embodiment, R₁ and R₂ are both chloro, R₅ is acetyl, and x is 1. In a further embodiment, R₁ is halo and R₂ is C₁₋₄ alkyl or C₁₋₄ haloalkyl.

In another embodiment, R₁ and R₂ are independently selected from the group consisting of hydrogen, chloro, fluoro, -CF₃, and -OCF₃, wherein at least one of R₁ and R₂ is not hydrogen.

In a further embodiment, R is methyl.

In another embodiment, the R₃ and R₄ are hydrogen and x = 1.

In another embodiment, phenyl ring A and B are not further substituted.

In an embodiment of the method, the compound of Formula XI is above 95% ee. In another embodiment, the compound of Formula XI is above 99% ee. In an embodiment, the compound of Formula XII is above 95% ee. In another embodiment, the compound of Formula XII is above 99% ee. In another embodiment, the compounds of Formula XI and XII are both above 99% ee. In another embodiment, the compounds of Formula XI and XII are both at about 100% ee.

In an embodiment, the above method further comprises separating the compound of hydroxy ester of Formula XI from acetyl ester of Formula XII. In an embodiment, the separation procedure uses chromatography. In a further embodiment, the separation procedure is flash chromatography. In a further embodiment, the compounds of Formula XI and XII may be transformed into compounds of Formula I, IA, or IB using standard chemical transformations known to one of ordinary skill in the art. In another embodiment, the transesterification reaction can be repeated to improve the optical purity of either the resulting alpha-hydroxy ester (Formula XI) or the resulting alpha-acyl ester (Formula XII).

In an alternate embodiment, the method above is used wherein Formula X is a compound of Formula XA: 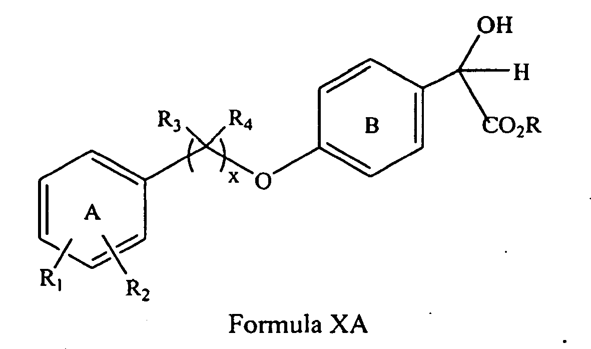 wherein all of R₃, R₄, R and x are defined as above for Formula X, and R₁ and R₂ are both halo. The halo is iodo, fluoro, chloro, or bromo. In an alternate embodiment, both R₁ and R₂ are chloro. In a further embodiment, R₁ is halo and R₂ is C₁₋₄ alkyl or C₁₋₄ haloalkyl. In an embodiment, x is 1 or 2. In a further embodiment, R₁ and R₂ are both chloro, and x is 1. In another embodiment, R₁ and R₂ are independently selected from the group consisting of hydrogen, chloro, fluoro, -CF₃, and -OCF₃, wherein at least one of R₁ and R₂ is not hydrogen. In a further embodiment, R is methyl. In another embodiment, the R₃ and R₄ are hydrogen and x = 1. In another embodiment, phenyl ring A and B are not further substituted.

In an alternate embodiment, the method above is used wherein Formula X is a compound of Formula XB: 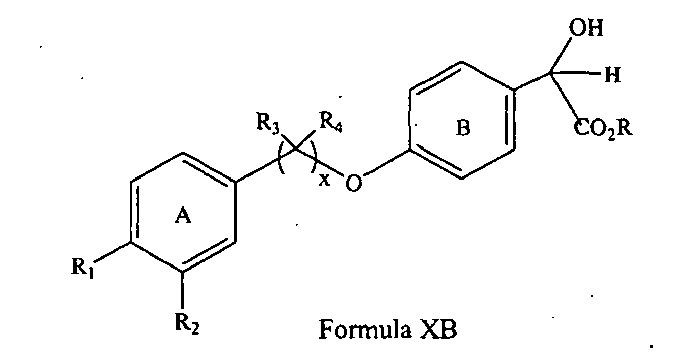 wherein all of R₃, R₄, R and x are defined as above for Formula X and R₁ and R₂ are both halo. The halo is iodo, fluoro, chloro, or bromo. In an alternate embodiment, both R₁ and R₂ are chloro. In a further embodiment, R₁ is halo and R₂ is C₁₋₄ alkyl or C₁₋₄ haloalkyl. In an embodiment, x is 1 or 2. In a further embodiment, R₁ and R₂ are both chloro, and x is 1. In a further embodiment, R₁ and R₂ are independently selected from the group consisting of hydrogen, chloro, fluoro, -CF₃, and -OCF₃, wherein at least one of R₁ and R₂ is not hydrogen. In a further embodiment, R is methyl. In another embodiment, the R₃ and R₄ are hydrogen and x = 1. In another embodiment, phenyl ring A and B are not further substituted.

In an embodiment of the method, the compound of Formula X, XA, or XB may be combined with an amount of solvent to improve its solubility or rate of dissolution in another solvent used in a reaction mixture. In an embodiment, a compound of Formula X, XA, or XB is combined with ethyl acetate to form a syrup and thereby improve its solubility in another reaction solvent such as diisopropyl ether.

The ester compounds that may be useful for transesterification include ethyl acetate, ethyl propionate, ethyl butyrate, ethyl stearate, trichloro-ethyl laurate, butyl laurate, ethylene glycol diacetate, triacetin, tripropionin, tributyrin, tricaproin, tristearin, trilaurin, trimyristin, triolein, and fatty acid vinyl esters such as vinyl acetate, vinyl caproate, and vinyl laurate. In an embodiment, the ester compound is vinyl acetate.

Any enzyme operable to transesterify an ester compound with a racemic compound of Formula X, XA, or XB may be used. For example, any of a number of lipases may be used in the above reaction. For example, the following lipases may be used: Lipase AP from *Aspergillus niger*, Lipase M from *Mucor javanicus*, Lipase P from *Pseudomonas fluorescens*, Lipase PS from *Pseudomonas fluorescens*, Lipase CES from *Pseudomonas sp*, Lipase CE from *Humicola lanuginosa*, Lipase AP from *Rhizopus javanicus*, Lipase II from *Porcine* Pancreas, Lipase VIII from *Geotrichum Candidum*, Lipase X from *Rhizopus delama*r, Lipase from *Chromobacterium Viscosum*, Palatase A from *Aspergillus niger*, Lipase from *Rhizopus niveus*, and Lipase B from *Pseudomonas fragi.* All of the above are available from Sigma Chemical Co. (St. Louis, Missouri) or Amano Pharmaceutical Co., Ltd. (Nagoya, Japan). It should be understood that other commercially available lipases or other lipases that can be purified can also be used in the present invention to generate the optically active pure compounds of the present invention. In an embodiment, the enzyme is a lipase and the lipase Amano PS from Psuedomonas Cepacia.

It should be understood that a plurality of different embodiments are shown and described above and below. It is contemplated and therefore within the scope of the present invention that any one or more feature that is on any one embodiment can be combined with any one or more feature from any other embodiment to generate a different embodiment that is not explicitly disclosed.

A generalized scheme of making the compounds of the present invention is shown in the below scheme. It should be recognized that the starting material in the below method can be modified so that any regiochemistry can be used. For example, the very first starting material in the scheme below (i.e., hydroxy(4-hydroxyphenyl)acetic acid) can have any of ortho, meta or para (as shown) substitution. This will allow the generation of compounds with different regiochemistries. Moreover, the 4-(bromomethyl)-1,2-dichlorobenzene compound shown in the scheme below can have any of a variety of regiochemistries allowing any of a plurality of regioisomers to be made using the below scheme or the comparable scheme with compounds that are regioisomers of those shown. Further, although the generalized scheme is shown with a 1,2 -dichloro substituted compound it should be recognized that other substitution patterns and other atoms (other than chloro) are possible and contemplated, and therefore within the scope of the present invention.

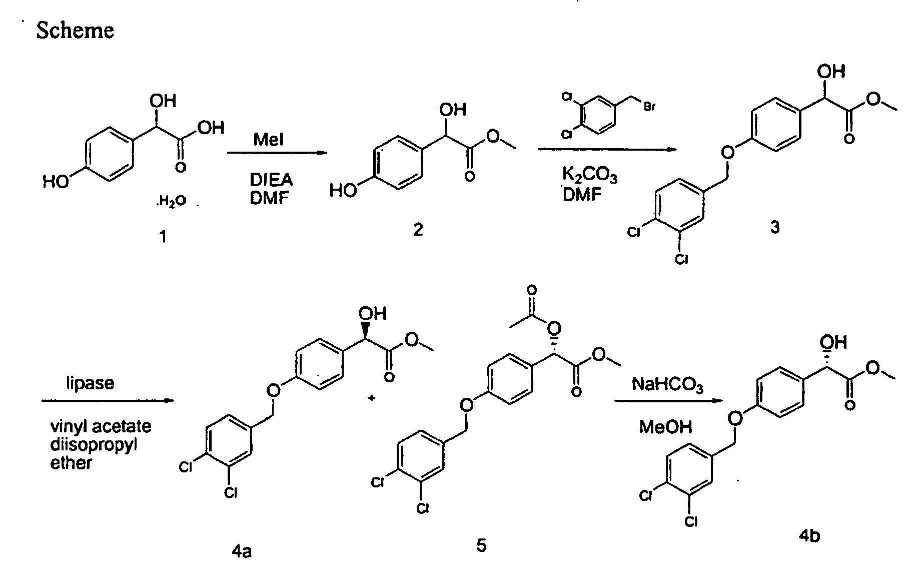

The lipase reaction in the above scheme may effectively generate one stereoisomer in about 100% ee relative to the other as the transesterification by the lipase only takes place for one of the enantiomers (and not the other). Further, the reaction may consume all of one enantiomer thereby providing a 50:50 mixture of alcohol 4a and acetyl ester 5. The different characteristics between the dissimilar chiral products (i.e, the resulting alcohol 4a and the acetyl ester 5) allow separation using flash chromatography or some other means of separating these products with different properties. After separation of the alcohol 4a using flash chromatography from the acetyl ester 5, the acetyl ester 5 can be hydrolyzed to give the opposite enantiomer 4b (in this instance, hydrolysis was performed using sodium bicarbonate in methanol) in high optical purity.

Advantages of the method described herein for preparing optically active α-hydroxy derivatives may include (1) little hydrolysis of esters because the transesterification reaction may be conducted under substantially anhydrous conditions; (2) the lipase may be recovered and re-used; and (3) the reaction can be performed under the conditions of relatively lower temperatures and an open system. Further, each antipode of the optically α-hydroxy derivatives may be obtained in high optical purity from one batch because the transesterification reaction may completely consume one enantiomer of the racemic mixture thereby potentially reducing waste, decreasing the time needed to prepare equal amounts of each enantiomer of a desired α-hydroxy derivative, and/or simplifying any purification processes.

### EXAMPLES:

### Example 1 - Synthesis of (±)-4-(3,4-dichlorobenzyloxy)mandelic acid methyl ester

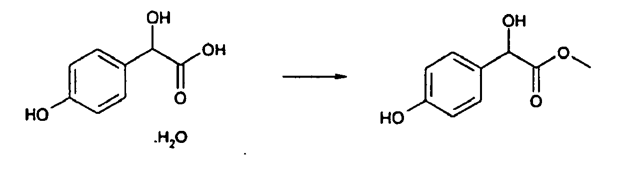

Step 1: To a solution of 4-hydroxymandelic acid monohydrate (18.16 g, 100 mmol) in anhydrous DMF (dimethyl formamide) (200 mL) was added DIEA (diisopropylethyl amine) (2.0 eq., 34.95 mL, 200 mmol), cooled to 0 °C in an ice bath and stirred at 0 °C for 10 minutes. Iodomethane (3.0 eq., 150 mL, 300 mmol, 2.0 M solution in tert-butylmethyl ether) was added at 0 °C. The reaction mixture was stirred for 24 h and allowed to warm up to room temperature while stirring. After completion of the reaction, the contents were concentrated in vacuum to remove excess DIEA, iodomethane, and tert-butylmethyl ether. The reaction mixture was subjected to next step without further purification. ,

LCMS: *m*/*z* 184 [M + 2]. ¹H NMR (400MHz, CDCl₃): δ 7.19 (d, 2H), 6.74 (d, 2H), 5.11 (s, 1H), and 3.72 (s, 3H).

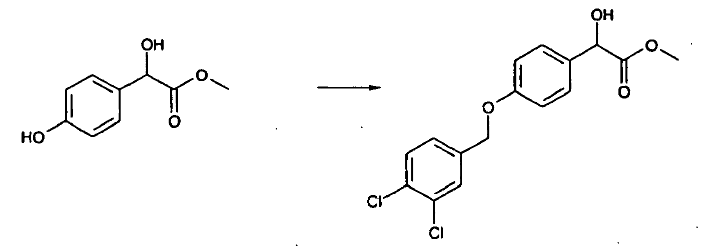

Step 2: 4-Hydroxymandelic acid methyl ester (18.21 g, 100 mmol) was redissolved in anhydrous DMF (100 mL) and potassium carbonate (2.0 eq., 27.6 g, 200 mmol) was added to the reaction mixture while stirring, then slowly 3,4-dichlorobenzylbromide (1.0 eq., 23.93 g, 100 mmol) was added to the reaction mixture in 10 minutes, and stirring was continued for 24 h. After completion of the reaction, ice-cold water (500 mL) was added to the reaction mixture and stirred for 16 hours. The precipitate was filtered off and washed with water (2 x 100 mL) and hexanes (3 x 200 mL) to give the crude product with a quantitative yield (34.2 g).

LCMS: *m*/*z* 342 [M + 2]. ¹H NMR (400MHz, CDCl₃): δ 7.53 (d, 1H), 7.45 (d, 1H), 7.35 (m, 1H), 7.33 (m, 1H), 7.25 (dd, 1H), 6.95 (m, 1H), 6.93 (m, 1H), 5.14 (s, 1H), 5.01 (s, 2H), and 3.76 (s, 3H).

### Example 2 - Resolution of (±)-4-(3,4-dichlorobenzyloxy)mandelic acid methyl ester

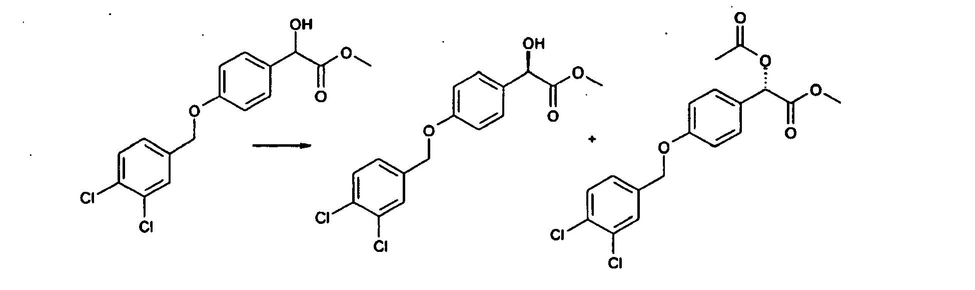

Step 1: (±)-[4-(3,4-Dichloro-benzyloxyl-phenyl]-hydroxy-acetic acid methyl ester (204 g, 0.6 mmol) was dissolved in diisopropyl ether (1200 mL), then vinyl acetate (3.0 eq., 166 mL, 1.8 mol) and lipase enzyme (113 g, Amano PS, from Pseudomonas Cepacia) were added to this solution. The reaction mixture was placed on a shaker. The reaction mixture was shaken at room temperature for 140 h. The reaction mixture was monitored by chiral HPLC after 90 hours of reaction time on a daily basis using Chiralpak AD-H column (90:10 ratio mixture of hexanes: isopropanol as an eluent system). After completion, the reaction mixture was filtered and concentrated to give the 1:1 mixture of (*R*)-[4-(3,4-dichloro-benzyloxy)-phenyl]-hydroxy-acetic acid methyl ester and (*S*)-acetoxy-[4-(3,4-dichloro-benzyloxy)-phenyl]-acetic acid methyl ester (181 g mixture, 89%). This crude mixture was separated by flash column silica gel chromatography using 90:10 to 70:30 hexanes: ethyl acetate as an eluent system to give (*R*)-[4-(3,4-dichloro-benzyloxy)-phenyl]-hydroxy-acetic acid methyl ester (85 g, 44.3%, 99.99% ee by chiral HPLC) LCMS: *m*/*z* 342 [M + 2]. ¹H NMR (400MHz, CDCl₃): δ 7.53 (d, 1H), 7.45 (d, 1H), 7.35 (m, 1H), 7.33 (m, 1H), 7.25 (dd, 1H), 6.95 (m, 1H), 6.93 (m, 1H), 5.14 (s, 1H), 5.00 (s, 2H), and 3.76 (s, 3H); HPLC: Chiralpak-AD-H Column, UV-254nm, hexanes: 2-propanol (90:10, v/v; 0.8 mL/min): (*S*)-isomer t_{R}: 27.1 min, (*R*)-isomer, t_{R}: 29.7 min and (*S*)-acetoxy-[4-(3,4-dichloro-benzyloxy)-phenyl]-acetic acid methyl ester (96 g, 44.3%, 99.99% by chiral HPLC), LCMS: *m*/*z* 384 [M + 2]. ¹H NMR (400MHz, CDCl₃): δ 7.53 (d, 1H), 7.46 (d, 1H), 7.39 (m, 1H), 7.33 (m, 1H), 7.24 (dd, 1H), 6.97 (m, 1H), 6.94 (m, 1H), 5.88 (s, 1H), 5.02 (s, 2H), 3.73 (s, 3H), and 2.19 (s, 3H), HPLC: Chiralpak-AD-H Column, UV-254nm, hexanes: 2-propanol (90:10, v/v; 0.8 mL/min): (*S*)-isomer t_{R}: 13.3 min, (*R*)-isomer t_{R}: 14.1 min).

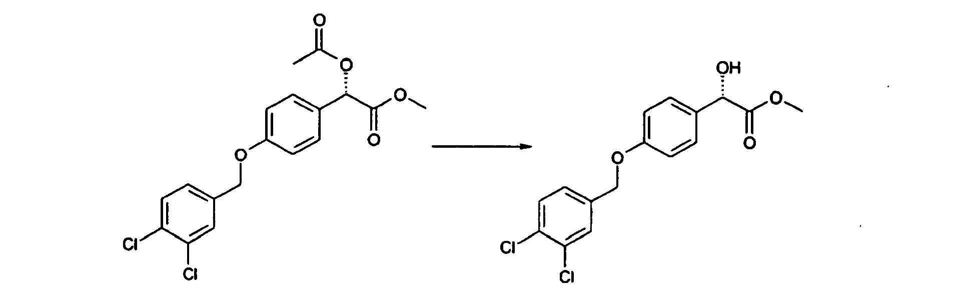

Step 2: To a solution of (*S*)-acetoxy-[4-(3,4-dichloro-benzyloxy)-phenyl]-acetic acid methyl ester (96 g, 251 mmol) in methanol (250 mL) was added sodium bicarbonate (3.0 eq., 63.3 g, 753 mmol) and stirred at room temperature for 16 hours. After completion of the reaction, the reaction mixture was filtered and concentrated under reduced pressure. The residue was acidified to pH 3-4 with dilute hydrochloric acid (1.0 N, 200 mL), and extracted with ethyl acetate (3 x 200 mL). The organic extracts were combined and washed with water (1 x 150 mL) and brine (2 x 150 mL), concentrated under reduced pressure to give (*S*)-[4-(3,4-dichloro-benzyloxy)-phenyl]-hydroxy-acetic acid methyl ester with a quantitative yield (85.4 g, 99.99% ee by chiral HPLC).

LCMS: *m*/*z* 342 [M + 2]. ¹H NMR (400MHz, CDCl₃): δ 7.53 (d, 1H), 7.45 (d, 1H), 7.35 (m, 1H), 7.33 (m, 1H), 7.25 (dd, 1H), 6.95 (m, 1H), 6.93 (m, 1H), 5.14 (s, 1H), 5.01 (s, 2H), and 3.76 (s, 3H). HPLC (Chiralpak-AD-H Column, UV-254nm, hexanes: 2-propanol (90:10, v/v; 0.8 mL/min): (*S*)-isomer t_{R}: 27.1 min, (*R*)-isomer t_{R}: 29.7 min).

### Example 3 - Synthesis of (R)- and (S)-4-hydroxymandelic acid methyl ester

In order to assign the absolute configuration of above enzymatic reaction products, each enantiomer was converted to known (R)- and (S)-enantiomers of 4-hydroxymandelic acid methyl esters and their retention times were compared on chiral HPLC with those reported (Tetrahedron Asymmetry, 16, 2005, p: 2113-2117). Both enantiomers were independently subjected to hydrogenation process by the following procedure.

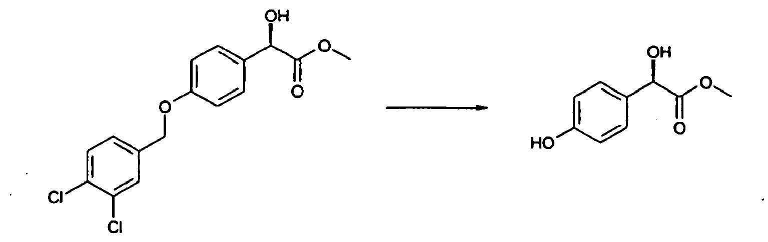

To a stirred solution of (*R*)-[4-(3,4-dichloro-benzyloxy)-phenyl]-hydroxy-acetic acid methyl ester (1.7 g, 5.0 mmol) in methanol mixture (50 mL) was added Pd/C (10 %, degussa type) and the resultant solution was degassed under reduced pressure subjected to hydrogenation at 40 - 50 psi of H₂ for 2 days. The catalyst was filtered through a pad of silica gel, washed the silica gel with methanol (25 mL) and ethyl acetate (25 mL). The combined filtrate was evaporated and purified with silica gel chromatography using hexanes: ethyl acetate (from 70:30 to 40:60) as an eluent system to give (*R*)-hydroxy-(4-hydroxy-phenyl)-acetic acid methyl ester (630 mg, 69.2%). LCMS: *m*/*z* 184 [M + 2]. ¹H NMR (400MHz, CDCl₃): δ 7.19 (d, 2H), 6.74 (d, 2H), 5.11 (s, 1H), and 3.73 (s, 3H). HPLC (Chiralpak-AD-H Column, UV-254nm, hexanes: 2-propanol (88:12, v/v; 0.8 mL/min): (*S*)-isomer t_{R}: 21.6 min, (*R*)-isomer t_{R}: 23.1 min (Ref.: Tetrahedron: Asymmetry, 16 (2005) p: 2113-2117).

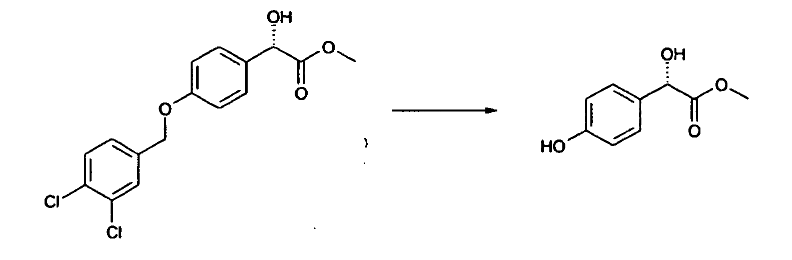

In the same manner, (*S*)-hydroxy-(4-hydroxy-phenyl)-acetic acid methyl ester (572 mg, 62.8%) was also isolated from (*S*)-[4-(3,4-dichloro-benzyloxy)-phenyl]-hydroxy-acetic acid methyl ester (1.7 gr, 5.0 mmol) from hydrogenolysis. LCMS: *m*/*z* 184 [M + 2]. ¹H NMR (400MHz, CDCl₃): δ 7.19 (d, 2H), 6.74 (d, 2H), 5.11 (s, 1H), and 3.72 (s, 3H). HPLC (Chiralpak-AD-H Column, UV-254nm, hexanes: 2-propanol (88:12, v/v; 0.8 mL/min): (*S*)-isomer t_{R}: 21.6 min, (*R*)-isomer t_{R}: 23.1 min) (Ref.: Tetrahedron: Asymmetry, 16 (2005) p: 2113-2117) .

### Example 4 - Synthesis of (±)-4-(3,4-dichtorobenzyloxy)mandelic acid methyl ester

Step 1: To a solution of 4-hydroxymandelic acid monohydrate (5000 g, 26.86 mol) in anhydrous dimethyl formamide (DMF) (18L) was added diisopropylethylamine (DIEA) (2.0 eq., 9.01 L, 54.91 mol) and stirred for 90 min at room temperature. Cooled to 0 °C in an ice bath and iodomethane (2.0 eq., 3.42 L, 54.91 mol) was added and stirred for 6h at 0 °C. Tert-butyl methyl ether (4 L) was added to the reaction mixture and allowed to warm-up to room temperature and stirred for 72 h. After completion of the reaction, solids were filtered and washed with DMF (4 L). The combined filtrate was concentrated in vacuum to remove excess DIEA, iodomethane, and tert-butyl methyl ether. The concentrated DMF solution was subjected to next step without further purification.

LCMS: *m*/*z* 184 [M + 2]. ¹H NMR (400MHz, CDCl₃): δ 7.19 (d, 2H), 6.74 (d, 2H), 5.11 (s, 1H), and 3.72 (s, 3H).

Step 2: Potassium carbonate (5670 g, 41.1 mol) was added to the DMF solution of 4-hydroxymandelic acid methyl ester while stirring. After 2h of stirring, the reaction mixture was cooled in an ice-bath, 3,4-dichlorobenzyl chloride (6400g, 32.74 mol) was added slowly to the reaction mixture in 1h. Potassium iodide (680 g, 4.11mol) was added and stirring was continued for 4-6 h at 0-10 °C. The reaction mixture was allowed to warm-up to room temperature and stirred for 48 h. After completion of the reaction, solids were filtered and washed with DMF (2 L). The filtrate was poured into ice-cold water (30 L) with stirring. Hexanes (4 L) were added to the suspension and stirring was continued for 8-10 hours. The precipitate was filtered and washed with water (6 L) and hexanes (4 L). The resulting solids were dissolved in minimum amount of CH₂Cl₂ (∼4 L). Hexanes (2 L) was added slowly to the solution of CH₂Cl₂ and stirred for 4-6 h. The resulting solids were filtered and dried to give the desired product (5700 g, 62% overall yield).

LCMS: *m*/*z* 342 [M + 2]. ¹H NMR (400MHz, CDCl₃): δ 7.53 (d, 1H), 7.45 (d, 1H), 7.35 (m, 1H), 7.33 (m, 1H), 7.25 (dd, 1H), 6.95 (m, 1H), 6.93 (m, 1H), 5.14 (s, 1H), 5.01 (s, 2H), and 3.76 (s, 3H).

### Example 5 - Resolution of (±)-4-(3,4-dichlorobenzyloxy)mandelic acid methyl ester

Step 1: (±)-[4-(3,4-Dichloro-benzyloxy)-phenyl]-hydroxy-acetic acid methyl ester (2500 g, 7.33 mol) was dissolved in ethyl acetate (6 L) and evaporated to a syrup (approximate volume 4 L). To this syrup, vinyl acetate (3.0 eq., 2 L, 22 mol) and diisopropyl ether (8 L), followed by lipase enzyme (1400 g, Amano PS, from Pseudomonas Cepacia) were added. The reaction mixture was stirred at room temperature for 8 days. The reaction mixture was monitored by chiral HPLC after 5 days of reaction time on a daily basis using Chiralpak OD-H column (90:10 ratio mixture of hexanes: isopropanol as an eluent system). After completion, the reaction mixture was filtered and concentrated to give the 3:2 mixture of (*R*)-[4-(3,4-dichloro-benzyloxy)-phenyl]-hydroxy-acetic acid methyl ester and (*S*)-acetoxy-[4-(3,4-dichloro-benzyloxy)-phenyl]-acetic acid methyl ester (2100 g mixture). This crude mixture was separated by flash column silica gel (∼10000 g) chromatography using 90:10 to 70:30 hexanes:ethyl acetate as an eluent system to give (*R*)-[4-(3,4-dichloro-benzyloxy)-phenyl]-hydroxyacetic acid methyl ester (1300 g, ∼80.0% ee by chiral HPLC) and and (*S*)-acetoxy-[4-(3,4-dichloro-benzyloxy)-phenyl]-acetic acid methyl ester (800 g, 96-98% ee). (*R*)-alcohol:

LCMS: *m*/*z* 342 [M + 2]. ¹H NMR (400MHz, CDCl₃): δ 7.53 (d, 1H), 7.45 (d, 1H), 7.35 (m, 1H), 7.33 (m, 1H), 7.25 (dd, 1H), 6.95 (m, 1H), 6.93 (m, 1H), 5.14 (s, 1H), 5.00 (s, 2H), and 3.76 (s, 3H); HPLC: Chiralpak-OD-H Column, UV-254nm, hexanes: 2-propanol (90:10, v/v; 0.8 mL/min): (*S*)-isomer t_{R}: 22.8 min, (*R*)-isomer, t_{R}: 42.8 min. (S)-Acetate:

LCMS: *mlz* 384 [M + 2]. ¹H NMR (400MHz, CDCl₃): δ 7.53 (d, 1H), 7.46 (d, 1H), 7.39 (m, 1H), 7.33 (m, 1H), 7.24 (dd, 1H), 6.97 (m, 1H), 6.94 (m, 1H), 5.88 (s, 1H), 5.02 (s, 2H), 3.73 (s, 3H), and 2.19 (s, 3H). HPLC: Chiralpak-AD-H Column, UV-254nm, hexanes: 2-propanol (90:10, v/v; 0.8 mL/min): (*S*)-isomer t_{R}: 13.3 min, (*R*)-isomer t_{R}: 14.1 min).

Step 2: To an ice-cold solution of (*S*)-acetoxy-[4-(3,4-dichloro-benzyloxy)-phenyl]-acetic acid methyl ester (800 g, 2.08 mol) in methanol (4L) was added sodium bicarbonate (2.5 eq., 438.3g, 5.22mol) and stirred at room temperature for 72 hours. After completion of the reaction, the reaction mixture was filtered and concentrated under reduced pressure. The residue was acidified to pH 3-4 with dilute hydrochloric acid (1.0 N, 4 L), and extracted with ethyl acetate (3 x 1.5 L). The organic extracts were combined and washed with water (1 x 2 L), brine (2 x 2 L), dried over sodium sulfate and concentrated under reduced pressure to give (*S*)-[4-(3,4-dichloro-benzyloxy)-phenyl]-hydroxy-acetic acid methyl ester in quantitative yield (648 g, 90% yield, >95% ee by chiral HPLC).

LCMS: *m*/*z* 342 [M + 2]. ¹H NMR (400MHz, CDCl₃): δ 7.53 (d, 1H), 7.45 (d, 1H), 7.35 (m, 1H), 7.33 (m, 1H), 7.25 (dd, 1H), 6.95 (m, 1H), 6.93 (m, 1H), 5.14 (s, 1H), 5.01 (s, 2H), and 3.76 (s, 3H). HPLC: Chiralpak-OD-H Column, UV-254nm, hexanes: 2-propanol (90: 10, v/v; 0.8 mL/min): (*S*)-isomer t_{R}: 22.8 min, (*R*)-isomer, t_{R}: 42.8 min.

It should be understood that the above examples are given only for the sake of showing that the reaction process can make a particular compound in the disclosed genus. The above procedure can be generalized so that all of the compounds in the disclosed genus can be made. Thus, the invention is not to be limited by the disclosed embodiment but rather is to be defined by the below claims.

## Claims

1. A compound of Formula I: 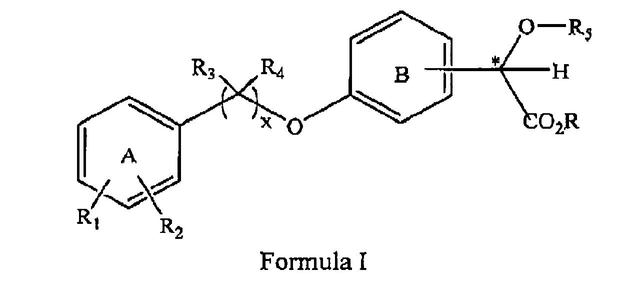 wherein
R₁ and R₂ are independently selected from the group consisting of: halo, hydroxy,
cyano, C₁₋₄ alkyl, C₁₋₄ alkoxy, C₁₋₄haloalkyl and C₁₋₄haloalkoxy, wherein at least one of R₁ and R₂ is halo,
R₃ and R₄ are each independently hydrogen, cyano, C₁₋₆ alkyl, C₁₋₆alkoxy, C₁₋₆
haloalkyl, C₁₋₆ haloalkoxy or phenyl, or taken together R₃ and R₄ are oxo ;
x is 1, 2, 3, 4, 5, or 6; ' R₅ is hydrogen or C₁₋₁₈ acyl;
wherein phenyl ring A and B are further optionally substituted with one or more of
halo, C₁₋₄ alkyl, C₁₋₄ alkoxy, C₁₋₄haloalkyl or C₁₋₄ haloalkoxy; and
R is hydrogen, C₁₋₆ alkyl, or an alkali metal ion.

2. The compound of Formula I of claim 1, wherein R₁ and R₂ are both halo.

3. The compound of Formula I of claim 1, wherein R₁ and R₂ are both chloro, R₅ is hydrogen or acetyl, and x is 1.

4. The compound of Formula I of claim 1, wherein R₁ is halo, and R₂ is C₁₋₄ alkyl or C₁₋₄ haloalkyl.

5. The compound of Formula I of claim 1, wherein R₁ and R₂ are independently selected from the group consisting of hydrogen, chloro, fluoro, - CF₃, and -OCF₃, wherein at least one of R₁ and R₂ is not hydrogen.

6. The compound of any one of claims 1 to 5, wherein R₃ and R₄ are hydrogen and x = 1.

7. The compound of any one of claims 1 to 6, wherein the compound of Formula I has the Formula IA:

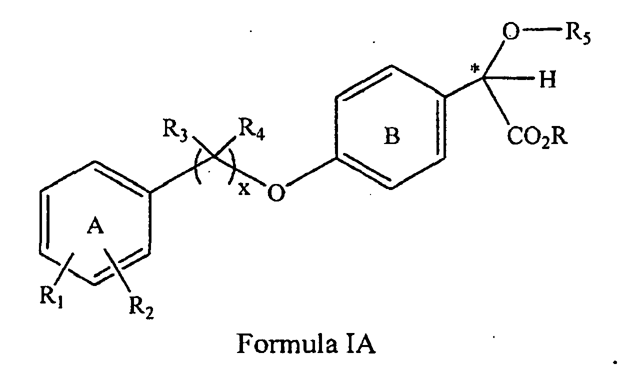

8. The compound of any one of claims 1 to 7, wherein the compound of Formula I has the Formula IB:

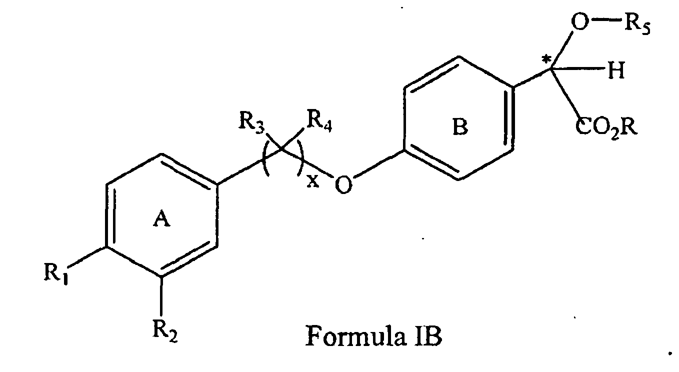

9. A method comprising reacting a compound of Formula X 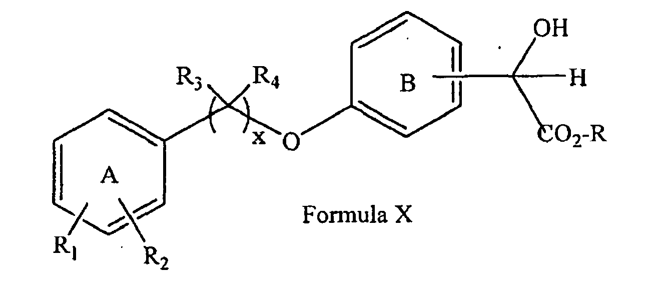 wherein
R₁ and R₂ are independently selected from the group consisting of: halo, hydroxy,
cyano, C₁₋₄ alkyl, C₁₋₆alkoxy, C₁₋₄haloalkyl and C₁₋₄ haloalkoxy, wherein at least one of R₁ and R₂ is halo,
R₃ and R₄ are each independently hydrogen, cyano, C₁₋₆ alkyl, C₁₋₆ alkoxy, C₁₋₆
haloalkyl, C₁₋₆ haloalkoxy or phenyl, or taken together R₃ and R₄ are oxo ;
x is 1, 2, 3, 4, 5, or 6;
wherein phenyl ring A and B are further optionally substituted with one or more of
halo, C₁₋₄ alkyl, C₁₋₄ alkoxy, C₁₋₄ haloalkyl or C₁₋₄ haloalkoxy; and
R is C₁₋₆ alkyl,
with an enzyme and an ester compound to form a reaction mixture;
generating a compound of Formula XI 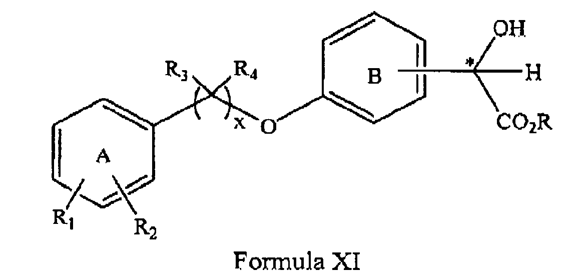 wherein R₁, R₂ R₃, R₄, R and x are as described above for the compound of Formula X, wherein the carbon with the * present is in the R or S configuration; and
generating a compound of Formula XII 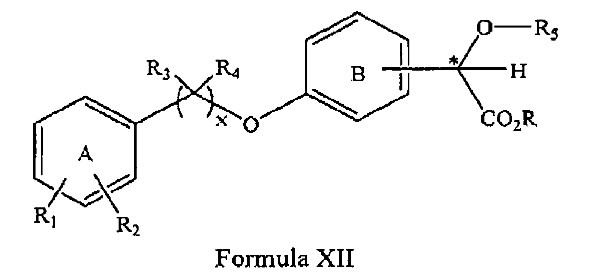 wherein R₁, R₂ R₃, R₄, R and x are as described above for the compound of Formula X and R₅ is a C₁₋₁₈ acyl group, and wherein the carbon with the * present is in the opposite configuration as the compound of Formula XI.

10. The method of claim 9, wherein R₁ and R₂ are both halo.

11. The method of claim 9, wherein R₁ and R₂ are both chloro, R₅ is acetyl, and x is 1.

12. The method of claim 9, wherein R₁ is halo, and R₂ is C₁₋₄ alkyl or C₁₋₄ haloalkyl.

13. The method of claim 9, wherein R₁ and R₂ are independently selected from the group consisting of hydrogen, chloro, fluoro, -CF₃, and -OCF₃, wherein at least one of R₁ and R₂ is not hydrogen.

14. The method of any one of claims 9 to 13, wherein the enzyme is a lipase.

15. The method of claim 14, wherein the lipase is Amano PS from Psuedomonas Cepecia.

## Patentansprüche

1. Verbindung der Formel I: 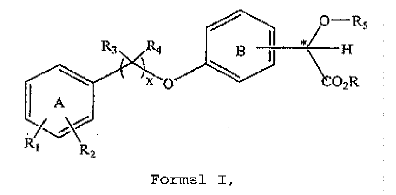 wobei
R₁ und R₂ unabhängig aus der Gruppe bestehend aus: Halogen, Hydroxy, Cyano, C₁₋₄-Alkyl, C₁₋₄-Alkoxy, C₁₋₄-Halogenalkyl und C₁₋₄-Halogenalkoxy ausgewählt sind, wobei mindestens eines von R₁ und R₂ Halogen ist,
R₃ und R₄ jeweils unabhängig Wasserstoff, Cyano, C₁₋₆-Alkyl, C₁₋₆-Alkoxy, C₁₋₆-Halogenalkyl, C₁₋₆-Halogenalkoxy oder Phenyl sind oder R₃ und R₄ zusammengenommen oxo sind;
x 1, 2, 3, 4, 5 oder 6 ist;
R₅ Wasserstoff oder C₁₋₁₈-Acyl ist;
wobei die Phenylringe A und B weiterhin gegebenenfalls mit einem oder mehreren von Halogen, C₁₋₄-Alkyl, C₁₋₄-Alkoxy, C₁₋₄-Halogenalkyl oder C₁₋₄-Halogenalkoxy substituiert sind; und
R Wasserstoff, C₁₋₆-Alkyl oder ein Alkalimetallion ist.

2. Verbindung der Formel I nach Anspruch 1, wobei R₁ und R₂ beide Halogen sind.

3. Verbindung der Formel I nach Anspruch 1, wobei R₁ und R₂ beide Chlor sind, R₅ Wasserstoff oder Acetyl ist und x 1 ist.

4. Verbindung der Formel I nach Anspruch 1, wobei R₁ Halogen ist und R₂ C₁₋₄-Alkyl oder C₁₋₄-Halogenalkyl ist.

5. Verbindung der Formel I nach Anspruch 1, wobei R₁ und R₂ unabhängig aus der Gruppe bestehend aus Wasserstoff, Chlor, Fluor, -CF₃ und -OCF₃ ausgewählt sind, wobei mindestens eines von R₁ und R₂ nicht Wasserstoff ist.

6. Verbindung nach einem der Ansprüche 1 bis 5, wobei R₃ und R₄ Wasserstoff sind und x = 1.

7. Verbindung nach einem der Ansprüche 1 bis 6, wobei die Verbindung der Formel I die Formel IA aufweist:

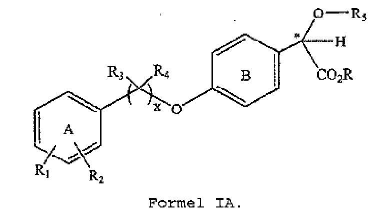

8. Verbindung nach einem der Ansprüche 1 bis 7, wobei die Verbindung der Formel I die Formel IB aufweist:

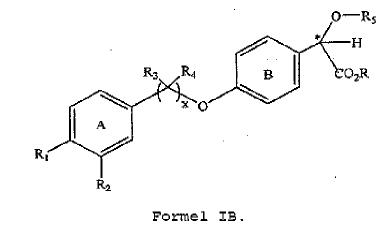

9. Verfahren, das das Umsetzen einer Verbindung der Formel X: 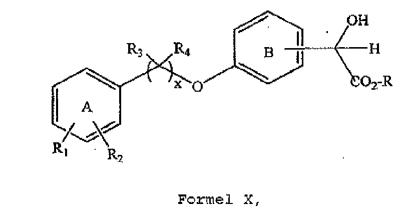 wobei
R₁ und R₂ unabhängig aus der Gruppe bestehend aus: Halogen, Hydroxy, Cyano, C₁₋₄-Alkyl, C₁₋₄-alkoxy, C₁₋₄-Halogenalkyl und C₁₋₄-Halogenalkoxy ausgewählt sind, wobei mindestens eines von R₁ und R₂ Halogen ist,
R₃ und R₄ jeweils unabhängig Wasserstoff, Cyano, C₁₋₆-Alkyl, C₁₋₆-Alkoxy, C₁₋₆-Halogenalkyl, C₁₋₆-Halogenalkoxy oder Phenyl sind oder R₃ und R₄ zusammengenommen Oxo sind;
x 1, 2, 3, 4, 5 oder 6 ist;
wobei die Phenylringe A und B weiterhin gegebenenfalls mit einem oder mehreren von Halogen, C₁₋₄-Alkyl, C₁₋₄-Alkoxy, C₁₋₄-Halogenalkyl oder C₁₋₄-Halogenalkoxy substituiert sind; und
R C₁₋₆-Alkyl ist,
mit einem Enzym und einer Esterverbindung, um ein Reaktionsgemisch zu bilden;
das Erzeugen einer verbindung der Formel XI: 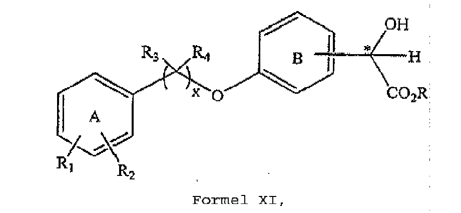 wobei R₁, R₂, R₃, R₄, R und x wie oben für die Verbindung der Formel X beschrieben sind, wobei der Kohlenstoff mit dem vorhandenen * in der R- oder S-Konfiguration ist; und das Erzeugen einer Verbindung der Formel XII: 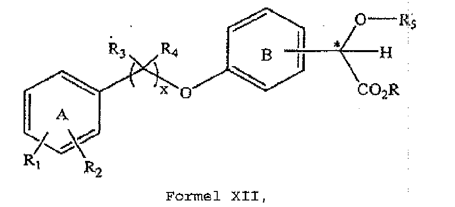 wobei R₁, R₂, R₃, R₄, R und x wie oben für die Verbindung der Formel X beschrieben sind und R₅ eine C₁₋₁₈-Acylgruppe ist und wobei der Kohlenstoff mit dem vorhandenen * in der entgegengesetzten Konfiguration zu der Verbindung der Formel XI ist, umfasst.

10. Verfahren nach Anspruch 9, wobei R₁ und R₂ beide Halogen sind.

11. Verfahren nach Anspruch 9, wobei R₁ und R₂ beide Chlor sind, R₅ Acetyl ist und x 1 ist.

12. Verfahren nach Anspruch 9, wobei R₁ Halogen ist und R₂ C₁₋₄-Alkyl oder C₁₋₄-Halogenalkyl ist.

13. Verfahren nach Anspruch 9, wobei R₁ und R₂ unabhängig aus der Gruppe bestehend aus Wasserstoff, Chlor, Fluor, -CF₃ und -OCF₃ ausgewählt sind, wobei mindestens eines von R₁ und R₂ nicht Wasserstoff ist.

14. Verfahren nach einem der Anspruche 9 bis 13, wobei es sich bei dem Enzym um eine Lipase handelt.

15. Verfahren nach Anspruch 14, wobei es sich bei der Lipase um Amano PS von Pseudomonas cepecia handelt.

## Revendications

1. Composé de Formule I : 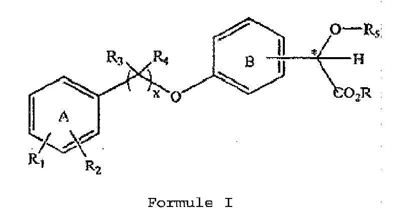 dans laquelle
R₁ et R₂ sont indépendamment sélectionnés parmi le groupe constitué de : halo, hydroxy, cyano, alkyle en C₁₋₄, alkoxy en C₁₋₄, haloalkyle en C₁₋₄ et haloalkoxy en C₁₋₄, dans laquelle au moins un parmi R₁ et R₂ est halo,
R₃ et R₄ sont chacun indépendamment hydrogène, cyano, alkyle en C₁₋₆, alkoxy en C₁₋₆, haloalkyle en C₁₋₆, haloalkoxy en C₁₋₆, ou phényle, ou pris ensemble R₃ et R₄ sont oxo ;
x est 1, 2, 3, 4, 5, ou 6 ;
R₅ est hydrogène ou acyle en C₁₋₈ ;
dans laquelle les noyaux phényles A et B sont davantage éventuellement substitués par un ou plusieurs parmi halo, alkyle en C₁₋₄, alkoxy en C₁₋₄, haloalkyle en C₁₋₄ ou haloalkoxy en C₁₋₄ ; et
R est hydrogène, alkyle en C₁₋₆, ou un ion de métal alcalin.

2. Composé de Formule I selon la revendication 1, dans lequel R₁ et R₂ sont tous les deux halo.

3. Composé de Formule 1 selon la revendication 1, dans lequel R₁ et R₂ sont tous les deux chloro, R₅ est hydrogène ou acétyle et x est 1.

4. Composé de Formule I selon la revendication 1, dans lequel R₁ est halo, et R₂ est alkyle en C₁₋₄ ou haloalkyle en C₁₋₄.

5. Composé de Formule 1 selon la revendication 1, dans lequel R₁ et R₂ sont indépendamment sélectionnas parmi le groupe constitué d'hydrogène, chloro, fluoro, -CF₃, et OCF₃, dans lequel au moins un parmi R₁ et R₂ n'est pas hydrogène.

6. Composé selon l'une quelconque des revendications 1 à 5, dans lequel R₃ et R₄ sont hydrogène et x = 1.

7. Composé selon l'une quelconque des revendications 1 à 6, dans lequel le composé de Formule I a la Formule IA ;

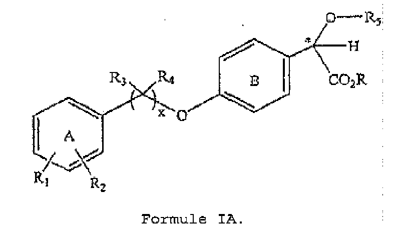

8. Composé selon l'une quelconque des revendications 1 à 7, dans lequel le composé de Formule I a la Formule IB :

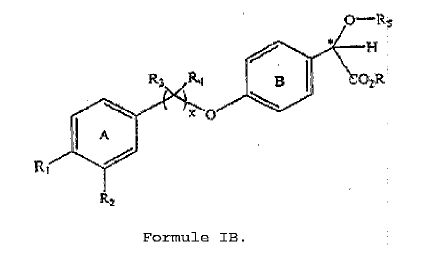

9. Procédé comprenant la réaction d'un composé de Formule X 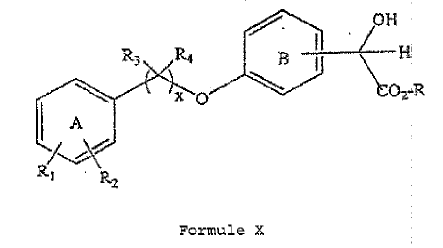 dans laquelle
R₁ et R₂ sont indépendamment sélectionnés parmi le groupe constitué de : halo, hydroxy, cyan, alkyle en C₁₋₄, alkoxy en C₁₋₄, haloalkyle en C₁₋₄ et haloalkoxy en C₁₋₄, dans laquelle au moins un parmi R₁ et R₂ est halo,
R₃ et R₄ sont chacun indépendamment hydrogène, cyano, alkyle en C₁₋₆, alkoxy en C₁₋₆, haloalkyle en C₁₋₆, haloalkoxy en C₁₋₆, ou phényle, ou pris ensemble R₃ et R₄ sont oxo ;
x est 1, 2, 3, 4, 5, ou 6 ;
dans laquelle les noyaux phényles A et B sont davantage éventuellement substitués par un ou plusieurs parmi halo, alkyle en C₁₋₄, alkoxy en C₁₋₄, haloalkyle en C₁₋₄ ou haloalkoxy en C₁₋₄ ; et
R est alkyl en C₁₋₆,
avec une enzyme et un composé ester pour former un mélange réactionnel ;
générant un composé de Formule XI 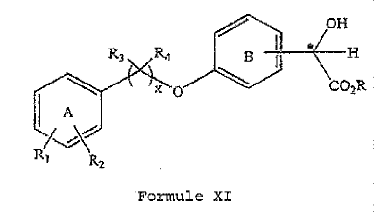 dans laquelle R₁, R₂, R₃, R₄, R et x sont tels que décrit ci-dessus pour le composé de Formule X, dans laquelle le carbone avec * présent est dans la configuration R ou S ;
et
générait un composé de Formule XII 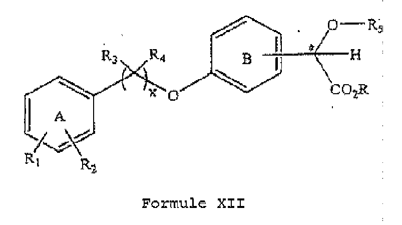 dans laquelle R₁, R₂, R₃, R₄, R et x sont tels que décrit ci-dessus pour le composé de Formule X et R₅ est un groupe acyle en C₁₋₁₈, et dans laquelle le carbone avec * présent est dans la configuration opposée à celle du composé de Formule XI.

10. Procédé selon la revendication 9, dans lequel R₁ et R₂ sont tous les deux halo.

11. Procédé selon la revendication 9, dans lequel R₁ et R₂ sont tous les deux chloro, R₅ est acétyle, et x est 1.

12. Procédé selon la revendication 9, dans lequel R₁ est halo, et R₂ est alkyle en C₁₋₄ ou haloalkyle en C₁₋₄.

13. Procédé selon la revendication 9, dans lequel R₁ et R₂ sont indépendamment sélectionnés parmi le groupe constitué d'hydrogène, chloro, fluoro, -CF₃, et -OCF₃, dans lequel au moins un parmi R₁ et R₂ n'est pas hydrogène.

14. Procédé selon l'une quelconque des revendications 9 à 13 dans lequel l'enzyme est une lipase.

15. Procédé selon la revendication 14, dans lequel la lipase est Amano PS de Psuedomonas Cepecia.
